# FASCICULE DE BREVET EUROPEEN

(11) **EP 1 797 164 B1**
(45) Date de publication et mention de la délivrance du brevet: **13.04.2016**
(21) Numéro de dépôt: 05779823.3
(22) Date de dépôt: 05.09.2005
(51) Int. Cl.: C12P 7/06, C02F 11/04, C10L 3/08, C12P 5/02

(54) **Procédé de production de carburant biologique**
Verfahren zur Herstellung von biologischem Brennstoff
Method for producing biological fuel

(30) Priorité: 06.09.2004 BE 200400432
(43) Date de publication de la demande: 20.06.2007
(73) Titulaire: Debailleul, Gerard, 7862 Ogy (BE); Van den Keybus, Eligius F. S., B-1500 Halle (BE)
(72) Inventeur: DEBAILLEUL, Gérard, B-7862 Ogy (BE)
(74) Mandataire: Claeys, Pierre
(86) Numéro de dépôt international: PCT/EP2005/054367
(87) Numéro de publication internationale: WO 2006/029971

(56) Documents cités:
- EP-A- 1 069 175
- DE-A1- 19 637 909
- DE-A1- 19 715 646
- TORRY-SMITH M ET AL: "Purification of bioethanol effluent in an UASB reactor system with simultaneous biogas formation." BIOTECHNOLOGY AND BIOENGINEERING, vol. 84, no. 1, 5 octobre 2003 (2003-10-05), pages 7-12, XP002384857 ISSN: 0006-3592

## Description

La présente invention est relative à un procédé de production de carburant biologique, comprenant
- une digestion en anaérobie de matière organique, avec formation d'un biogaz, et
- une production d'électricité et de chaleur par combustion du biogaz, avec prélèvement au moins partiel de cette électricité, pour alimenter en énergie au moins une étape du procédé consommant de l'électricité, et de cette chaleur, pour la transférer vers au moins une étape du procédé consommant de la chaleur (voir DE-A-19715646).

On connaît déjà des procédés de production d'éthanol à partir d'une biomasse, en particulier pour la production de carburant "vert" ou pour la formation d'un additif à introduire dans du carburant appelé ETBE (voir notamment JP 2004/141134, EP-A-1130085, US-A-4.952.504,US-A-5.733.758, US-A-5.837.506 et US-A-2002/0155583).

La production d'éthanol se fait à l'heure actuelle dans des industries classiques qui sont handicapées par un besoin énergétique important. Pour fabriquer de l'éthanol, il faut entre 60 et 80 % d'équivalent pétrole, ce qui ne contribue pas à une évolution vers l'utilisation d'un carburant vert à base d'éthanol pouvant être substitué aux carburants fossiles.

La fabrication d'éthanol est actuellement réservée à de grands producteurs, qui n'emploient pour cela que des matières premières riches en sucres et amidon, par exemple des betteraves sucrières, du blé, du maïs, de la canne à sucre. Pour pouvoir faire de l'éthanol dans des conditions économiques, souvent avec des interventions et un soutien indispensable des fonds publics, il est essentiel pour les distilleries actuelles de sélectionner de telles matières premières très riches, pour en tirer un maximum de rentabilité, rendre viable l'exploitation de transformation et compenser le coût élevé d'énergie fossile consommée. Ces matières premières sont toutefois saisonnières et ne permettent leur traitement qu'à une période déterminée, limitée, de l'année.

Par ailleurs, le monde agricole est entièrement soumis aux règles imposées de prix et de quotas, ainsi qu'aux coûts dus aux transports depuis l'exploitation jusqu'aux industries de traitement. L'agriculture devient une corporation qui doit être soutenue en permanence et la production des surplus agricoles ne fait que provoquer une chute des prix et donc directement une baisse des revenus des agriculteurs. Il est donc souhaitable de trouver des mesures permettant d'éviter que les pays industrialisés ne deviennent une grande jachère, ce qui serait une catastrophe écologique.

On connaît aussi un procédé complexe et énergivore de traitement de vieux bois comportant une fermentation éthanolique et une production de biogaz (voir DE-A-19637909).

La présente invention a pour but de mettre au point un procédé de production de carburant biologique qui surmonte les inconvénients précités, tout en permettant d'apporter au monde agricole une alternative d'exploitation de ses productions, et cela sans entraîner simultanément de pollution de l'environnement.

On a résolu ces problèmes suivant l'invention par un procédé de production de carburant vert biologique qu'indiqué au début, qui comprend en outre
- une fermentation éthanolique de matières végétales à tubercules et/ou herbacées et/ou de déchets végétaux de cultures agricoles,
- une séparation, à partir des matières végétales fermentées susdites, entre une suspension de matière organique à soumettre à ladite digestion en anaérobie et une phase liquide aqueuse contenant de l'éthanol, et
- une distillation de la phase liquide aqueuse contenant de l'éthanol avec récolte séparée d'eau et d'une phase éthanolique, qui forme le carburant biologique produit,
- ladite production d'électricité et/ou de chaleur ayant lieu par combustion uniquement dudit biogaz avec alimentation en électricité de toutes les étapes du procédé consommant de l'électricité et avec alimentation en chaleur de toutes les étapes du procédé consommant de la chaleur.

Le procédé suivant l'invention récupère donc les résidus organiques issus de la fermentation pour produire du biogaz qui va satisfaire les besoins énergétiques des étapes du procédé. Il s'est avéré en fait que la production d'électricité obtenue par ce biogaz dépasse les besoins en électricité du procédé et permet donc d'envisager en outre une injection d'électricité vers le réseau de distribution public. D'autre part, la chaleur cogénérée peut être récupérée au moins partiellement et elle est tout à fait suffisante pour satisfaire les besoins en chaleur de toutes les étapes du procédé. Les étapes nécessitant une consommation en électricité sont par exemple celles faisant intervenir des appareillages de broyage, notamment des matières végétales avant ladite fermentation, de pompage, par exemple pour les transferts de matière d'étape en étape, de mélange, comme par exemple le mélange de la suspension de matière organique au cours de la digestion, ou encore de séparation, par exemple dans des appareils de centrifugation. Les étapes nécessitant une consommation de chaleur peuvent par exemple être une hydrolyse catalytique des matières végétales avant fermentation, la distillation, la digestion, ainsi que d'autres étapes éventuelles, comme une pervaporation du distillat.

L'invention permet ainsi l'intégration de deux techniques connues qui sont la production de bioéthanol et celle de biogaz. Le procédé suivant l'invention produit ainsi, par la combinaison de ces productions, du carburant vert sous deux formes, sans apport d'énergie extérieure, ni introduction d'eau de distribution.

Le procédé peut donc être totalement autosuffisant en matière de consommation d'énergie, et il permet donc son utilisation avec des matières végétales variées qui sont produites de manière équilibrée pendant une grande partie de l'année.

Par matières végétales suivant l'invention, il faut entendre non seulement des déchets végétaux, mais aussi des produits de culture ou encore des végétaux sauvages. L'invention permet ainsi de remettre en question la façon de cultiver et d'organiser les surfaces de culture, en réservant une partie de l'exploitation à la culture de plantes énergétiques, de préférence celles ne nécessitant pas d'outillage particulièrement coûteux ou l'intervention de sociétés spécialisées pour les récoltes. On peut citer à titre d'exemple des herbacées, telles que les herbes de type Ray Gras, le miscanthus (herbe à éléphant) ou certaines essences à tubercule, comme le topinambour. Ces herbes de jachères et ou essences à tubercules contiennent du sucre, de l'amidon ou de la cellulose. La présente invention permet de récupérer de l'éthanol dans ces matières qui ne pourraient pas être acceptées dans les industries actuelles. Elle permet aussi aux exploitants agricoles de trouver une source de revenu par une valorisation de leur production, différente de celle actuellement utilisée.

Le procédé permet la production d'un carburant vert, l'éthanol, et l'exploitation d'une énergie verte, issue de la combustion d'un autre carburant vert, le biogaz. Il n'y a aucun rejet toxique dans l'atmosphère, le CO₂ rejeté n'est qu'un retour du captage de CO₂ par les plantes traitées. Le résidu de la digestion contient les sels NPK utilisables avantageusement sous la forme d'un engrais organique équilibré pour la fertilisation par épandage sur des terres agricoles, ce qui n'est qu'une restitution au sol du prélèvement des plantes.

Lé procédé suivant l'invention permet, par sa conception intégrée de deux techniques, d'envisager l'implantation du matériel nécessaire à la mise en oeuvre de ce procédé dans des sites agricoles de petite ou moyenne capacité. En effet le procédé permet un traitement rapide, non coûteux, de matières végétales très périssables et intransportables étant donné la vitesse à laquelle elles fermentent. Le procédé permet donc une meilleure rentabilité par une extraction plus complète de la chaîne carbonée des végétaux.

L'invention permet aussi de modifier les plans de culture, en utilisant des essences de plantes qui ne nécessitent ni engrais minéraux, ni herbicides, pesticides et autres produits phytosanitaires toujours polluants, en particulier pour la nappe phréatique. Ainsi le cultivateur n'investira pas inutilement dans des applications de ces produits coûteux.

Enfin, les pays du Sud jouissant d'un climat tropical ou subtropical bénéficient d'une végétation abondante et propice à la mise en oeuvre de la présente invention. En effet ce procédé peut être réalisé même dans des régions non desservies en énergie électrique et permet de remédier à des conditions d'accès difficile pour l'acheminement de carburant fossile. La présente invention permettrait de proposer ainsi à des populations défavorisées une amélioration nette des conditions de vie.

Suivant un mode de réalisation de l'invention, le procédé comprend, entre ladite étape de fermentation et ladite étape de distillation, une période de stockage desdites matières végétales fermentées et/ou de ladite phase liquide aqueuse contenant de l'éthanol. Comme les produits issus de la fermentation sont stables, on peut envisager de les stocker, notamment dans des réservoirs ordinaires, pour les distiller d'une manière étalée dans le temps, ce qui permet de poursuivre la production d'éthanol et de biogaz également dans les périodes hivernales.

Suivant un mode avantageux de réalisation de l'invention, le procédé comprend en outre une exposition à une osmose inverse de ladite phase liquide aqueuse contenant de l'éthanol, avec obtention d'une phase à particules solides, éventuellement recyclée à ladite étape de séparation, et d'une phase liquide épurée contenant de l'éthanol qui est transférée à ladite étape de distillation. Au cours de cette osmose, la phase liquide aqueuse contenant de l'éthanol est épurée et il en résulte que, lors de la distillation, l'eau séparée est très pure. Elle peut ainsi être au moins partiellement utilisée dans des étapes du procédé nécessitant un apport d'eau. On peut par exemple prévoir, avant ou pendant la digestion, une dilution par de l'eau de la suspension de matière organique issue de l'étape de séparation et rendre le procédé indépendant de l'alimentation en eau du réseau. L'eau en surplus ainsi traitée peut aussi être évacuée sans problème car elle répond aux normes imposées en matière de pureté.

Suivant un mode de réalisation perfectionné de l'invention, le procédé comprend en outre, après ladite étape de distillation, une pervaporation de ladite phase éthanolique distillée comportant une vaporisation d'éthanol au travers d'au moins une membrane et une rétention d'eau par ladite membrane. Cette étape permet avantageusement de raccourcir drastiquement la longueur de la colonne de distillation et de réaliser ainsi le procédé dans un équipement compact qui peut être mis en oeuvre dans une exploitation agricole ou dans une coopérative de petite ou moyenne dimension. On peut même envisager le transport de l'équipement de distillation, de pervaporation, de formation du biogaz et de transformation de celui-ci en énergie sur une ou plusieurs plates-formes véhiculables, et cela d'une exploitation agricole à l'autre.

On peut aussi prévoir, suivant l'invention, d'ajouter à l'étape de digestion de la suspension de matière organique, issue de l'étape de séparation, des matières organiques d'origine différente, par exemple des déchets organiques ménagers, des effluents d'animaux, de la boue biologique, etc.

D'autres modes de réalisation de l'invention sont indiqués dans les revendications annexées.

D'autres détails et particularités de l'invention vont à présent être décrits de manière plus détaillée à l'aide d'un exemple de réalisation d'installation intégrée mettant en oeuvre un procédé suivant l'invention.

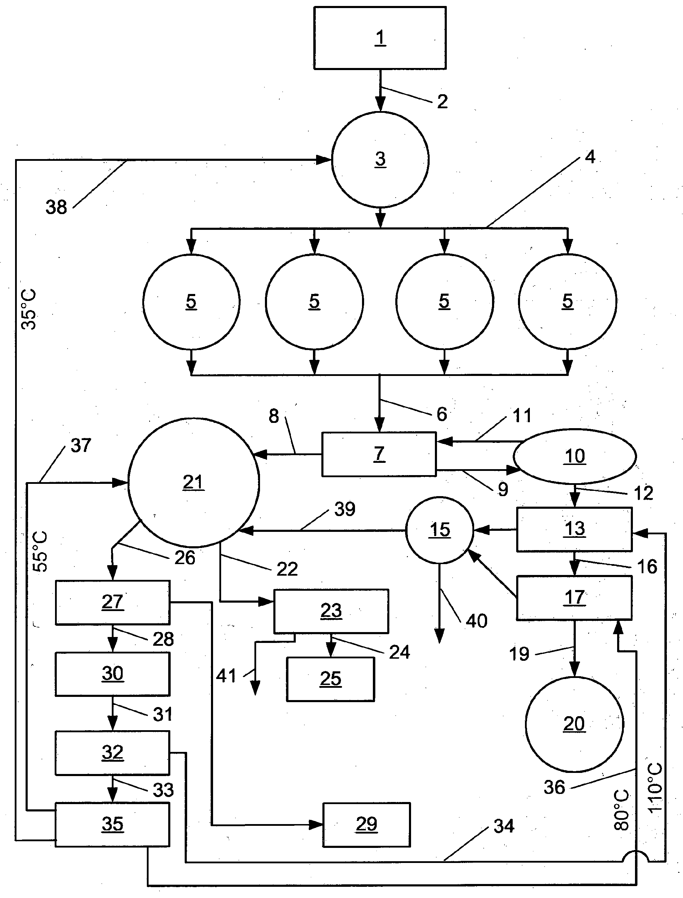

La figure annexée représente de manière schématique une telle installation.

Sur la figure unique annexée, l'installation comprend d'une manière intégrée un module de production d'éthanol et un module de production de biogaz, en particulier de biométhane.

Les matières premières d'origine végétale sont avantageusement préalablement broyées dans un broyeur 1 de manière à obtenir une pulpe ayant une taille de particule relativement fine. Cette pulpe est ensuite de préférence transférée par le conduit 2 dans un réacteur d'hydrolyse 3. Ce réacteur contient des enzymes usuelles appropriées pour obtenir une hydrolyse en présence de bactéries de préférence mésophiles. La température est de l'ordre de 15 à 35°C, de préférence de 35°C. Après hydrolyse, une fermentation est démarrée dans le réacteur 3 avec des levures usuelles, par exemple celles utilisées en brasserie. Cette fermentation initiale démarre à la température ambiante et est exothermique, elle dure 2 à 4 jours selon les matières premières. La fermentation est ensuite poursuivie, dans l'exemple de réalisation illustré, dans plusieurs cuves de fermentation 5, quatre en particulier, où le produit de fermentation initiale est transféré par le conduit 4 et séjourne pendant 8 à 12 jours selon la matière première. Après cette période de temps, la totalité de l'éthanol que l'on peut obtenir à partir du procédé suivant l'invention est formé et se trouve dans la pulpe fermentée qui est un produit stable et qui peut éventuellement être entreposée d'une manière non représentée avant de poursuivre son traitement.

Par le conduit de transfert 6, la pulpe fermentée est alimentée à partir des cuves de fermentation 5, en alternance, à un dispositif de séparation 7. Dans l'exemple illustré, le dispositif de séparation est une centrifugeuse. On pourrait bien entendu faire usage d'autres types de séparateur, par exemple un filtre-presse, une bande filtrante, etc. Dans le dispositif de séparation, on sépare, d'une part, une phase liquide aqueuse contenant de l'éthanol et, d'autre part, une suspension de matière organique d'aspect pâteux qui peut par exemple contenir jusqu'à 30 % de matière sèche.

La phase liquide contenant l'éthanol est transférée avantageusement, par un conduit 9, dans un dispositif d'osmose inverse, de préférence à membranes vibrantes. Une phase à particules solides est séparée par l'osmose et est par exemple recyclée au dispositif de séparation 7 par le conduit 11. La phase épurée par l'osmose est constituée principalement, sinon exclusivement, d'un mélange d'éthanol et d'eau. Ce liquide est transféré par le conduit 12 à une colonne de distillation 13 qui, dans l'exemple de réalisation illustré, est simple et courte. La distillation est effectuée à une température supérieure au point d'ébullition de l'éthanol (78,35°C). On obtient ainsi, au bas de la colonne, de l'eau qui peut être conduite vers un réservoir à eau 15 et, au haut de la colonne, de l'éthanol à un degré de pureté de l'ordre de 90 à 95 %.

Avantageusement l'éthanol distillé est transféré par un conduit 16 vers un dispositif de pervaporation 17 connu en soi. Ici l'éthanol, amené à nouveau sous forme de vapeur, est passé au travers de membranes denses, par exemple en matière céramique, l'eau étant retenue par les membranes. Dans ce cas, l'éthanol produit peut atteindre un degré de pureté supérieur à 95 %, par exemple de l'ordre de 98 à 99,9 %, ce qui est un produit économiquement intéressant. L'eau séparée au cours de la pervaporation est transférée au réservoir à eau 15 tandis que l'éthanol est transféré par le conduit 19 à un réservoir à éthanol 20.

Comme on peut le voir sur la figure annexée, une cuve de digestion 21 est alimentée en matières organiques qui, suivant l'invention, sont constitués, dans l'exemple illustré, exclusivement de la suspension de matière organique issue du séparateur 7. Cette suspension est acheminée par des moyens de transport 8 dans la cuve de digestion 21. Ces moyens de transport sont connus et peuvent être par exemple un conduit dans le cas où la suspension de matière organique est capable de s'écouler, une bande transporteuse lorsque la suspension est trop visqueuse, ou tout autre moyen approprié quelconque. D'une manière usuelle la cuve de digestion est avantageusement pourvue d'un mélangeur. Elle peut être alimentée en eau, notamment à partir du réservoir à eau 15, par l'intermédiaire du conduit 39. L'apport d'eau peut aussi se faire déjà à la sortie du séparateur 7, dans les moyens de transport 8 de la suspension de matière organique.

On peut aussi prévoir d'ajouter à la biomasse contenue dans la cuve de digestion des matières organiques d'une origine différente, sans que cela soit critique pour l'invention.

La digestion s'effectue en anaérobie en présence de bactéries thermophiles usuelles, par exemple à une température de 50 à 70°C, de préférence de l'ordre de 55°C, pendant une durée de séjour d'au moins 10 jours.

Le résidu de la digestion est constitué d'un milieu aqueux contenant de faibles teneurs en matières sèches, principalement inorganiques. Ce milieu aqueux peut avantageusement être transféré par le conduit 22 à un dispositif de traitement 23 ultérieur. Ce traitement peut par exemple être une séparation, notamment une centrifugation. La phase solide est alors transférée par les moyens de transport 24 dans un silo de stockage d'engrais 25 qui ne présente pas d'odeurs désagréables ni prolifération d'insectes. Par la suite ce résidu pourra avantageusement être répandu sur les sols de culture. La phase liquide centrifugée est de l'eau qui est évacuée par le conduit de sortie 41. Cette eau est d'une qualité appropriée pour être utilisée par exemple comme liquide fertilisant d'arrosage dans les cultures ou les prairies, ou elle peut même éventuellement être évacuée dans le réseau public.

Le biogaz, issu de la cuve de digestion, contient généralement principalement du méthane. Il est, dans le cas illustré, transféré par le conduit 26 à un générateur d'électricité 27 usuel, adapté pour transformer l'énergie dégagée par la combustion du biogaz en électricité et en chaleur. Ce générateur peut par exemple être un moteur-alternateur muni d'un radiateur, dans lequel a lieu la combustion par explosion du biogaz.

L'électricité produite permet d'alimenter complètement en électricité tous les éléments de l'installation qui consomment de l'électricité, notamment le broyeur 3, la centrifugeuse du séparateur 7, le mélangeur de la cuve de digestion 21, la centrifugeuse du séparateur 23, l'éventuel moteur d'entraînement de la bande transporteuse des moyens de transport 8, etc. L'électricité excédentaire produite peut être vendue au réseau de distribution local ou international 29 ou selon les particularités des certificats verts.

L'eau chaude provenant du radiateur du générateur 27 est transférée à une chaudière à eau 30 dans laquelle a lieu un échange de chaleur avec les gaz de fumée, issus de la combustion du biogaz, en donnant lieu à une fabrication de vapeur d'eau. La vapeur d'eau est transférée par le conduit 31 à un module d'aménagement de la température de la vapeur d'eau 32, qui ajuste cette température aux besoins, par exemple à 110°C.

Par le conduit 34 cette vapeur d'eau à 110°C est alimentée à la colonne de distillation 13 pour chauffer celle-ci à la température de distillation appropriée, souhaitée.

De la vapeur d'eau peut aussi être transférée par le conduit 33 à un échangeur de chaleur 35 qui permet d'obtenir notamment de l'eau chaude à 80°C alimentée par le conduit 36 pour chauffer le dispositif de pervaporation 17, de l'eau chaude à 55°C alimentée par le conduit 37 pour chauffer la cuve de digestion 21, et de l'eau chaude à 35°C alimentée par le conduit 38 pour chauffer le réacteur d'hydrolyse 3. Après chauffage de ces différents dispositifs, l'eau refroidie, issue des échanges thermiques, est ramenée, d'une manière non représentée, à la chaudière 30, au module 32 et/ou à l'échangeur de chaleur 35, de manière à former un circuit d'eau fermé.

L'eau excédentaire du réservoir 15, qui est extrêmement pure, peut être évacuée en 40 en vue d'une utilisation ultérieure quelconque. Elle peut aussi être éliminée dans le réseau public.

On va indiquer ci-dessous quelques exemples où le procédé suivant l'invention peut avantageusement être mis en oeuvre.

Si du blé est semé en tant que culture destinée à produire de l'énergie, il ne faut pas attendre que le blé soit mûr. Il est préférable de le récolter à l'état vert, ce que les agriculteurs appellent à l'état pâteux, la production d'éthanol se faisant sur toute la plante, ce qui n'est pas possible avec la paille, lorsque seul le grain est traité pour une fabrication d'éthanol.

Il ne faut pas d'engins coûteux pour ce type de récolte de la plante entière, cette récolte prématurée permet de replanter certaines essences pour des récoltes d'automne, les conditions climatiques ayant moins d'influence sur la réussite économique de la culture.

Il en est de même pour les autres types de céréales, notamment pour le maïs.

On peut noter que des céréales détériorées avant mûrissement, par exemple pour des raisons climatiques, peuvent malgré tout, grâce au procédé suivant l'invention, être rentabilisées.

La betterave sucrière pour être acceptée en sucrerie ou distillerie doit être séparée du sommet du tubercule et du feuillage, ce que les agriculteurs appellent les collets. Cette partie de la betterave est perdue et abandonnée sur les champs, alors qu'elle représente plus de 15 % de la plante. Avec la présente invention cette partie du végétal est nettement mieux valorisée que dans l'usage actuel sous forme de fertilisant organique.

Les orties, chardons et mauvaises herbes de prairie peuvent être récupérés, alors qu'actuellement ils sont abandonnés et perdus.

Toutes les herbes de bord de route peuvent être traitées suivant l'invention alors qu'elles sont orientées actuellement vers le compostage, avec comme conséquence un dégagement de méthane dans l'atmosphère, qui est la première cause du trou de la couche d'ozone.

En cas de maladie des végétaux cultivés, par exemple des pommes de terre, les cultivateurs pourraient récupérer une partie de la valeur de leurs cultures perdues par l'application du procédé suivant l'invention sur les déchets végétaux résultant de cette maladie.

Il doit être entendu que la présente invention n'est en aucune façon limitée au mode de réalisation décrit ci-dessus et que bien des modifications peuvent y être apportées sans sortir du cadre des revendications annexées.

On peut par exemple aussi prévoir de prélever une partie de la vapeur et de l'eau chaude directement du radiateur du générateur 27, l'autre portion étant fournie par la chaudière 30.

Comme matières végétales, on utilise toutes matières d'origine végétale contenant des sucres, de l'amidon et/ou de la cellulose, produites ou obtenues en n'importe quelle saison. On peut par exemple envisager des produits ou déchets de betteraves, de pommes de terre, de maïs, de canne à sucre, de topinambour, d'endives, d'herbe, etc.

## Revendications

1. Procédé de production de carburant biologique, comprenant
- une digestion en anaérobie de matière organique, avec formation d'un biogaz, et
- une production d'électricité et de chaleur par combustion du biogaz, avec prélèvement au moins partiel de cette électricité, pour alimenter en énergie au moins une étape du procédé consommant de l'électricité, et de cette chaleur, pour la transférer vers au moins une étape du procédé consommant de la chaleur,
**caractérisé en ce qu'**il comprend en outre
- une fermentation éthanolique de matières végétales à tubercules et/ou herbacées et/ou de déchets végétaux de cultures agricoles,
- une séparation, à partir des matières végétales fermentées susdites, entre une suspension de matière organique à soumettre à ladite digestion en anaérobie et une phase liquide aqueuse contenant de l'éthanol, et
- une distillation de la phase liquide aqueuse contenant de l'éthanol avec récolte séparée d'eau et d'une phase éthanolique, qui forme le carburant biologique produit,
- ladite production d'électricité et de chaleur ayant lieu par combustion uniquement dudit biogaz avec alimentation en électricité de toutes les étapes du procédé consommant de l'électricité et avec alimentation en chaleur de toutes les étapes du procédé consommant de la chaleur.

2. Procédé suivant la revendication 1, **caractérisé en ce qu'**il ne comprend pas d'introduction d'eau de distribution dans le procédé.

3. Procédé suivant l'une des revendications 1 et 2, **caractérisé en ce que**, comme matières végétales à tubercules et/ou herbacées et/ou déchets végétaux de cultures agricoles, on fait usage des matières contenant des sucres, de l'amidon et/ou de la cellulose, en particulier de betteraves, de collets de betteraves, de pommes de terre, de topinambours, de céréales, notamment de blé et de maïs, d'orties, de chardons, de mauvaises herbes, de canne à sucre, d'endives, de miscanthus, d'herbes de jachères ou de leurs mélanges.

4. Procédé suivant l'une quelconque des revendications 1 à 3, **caractérisé en ce qu'**il comprend, entre ladite étape de fermentation et ladite étape de distillation, une période de stockage desdites matières végétales fermentées et/ou de ladite phase liquide aqueuse contenant de l'éthanol.

5. Procédé suivant l'une quelconque des revendications 1 à 4, **caractérisé en ce qu'**il comprend en outre une exposition à une osmose inverse de ladite phase liquide aqueuse contenant de l'éthanol, avec obtention d'une phase à particules solides, éventuellement recyclée à ladite étape de séparation, et d'une phase liquide épurée contenant de l'éthanol qui est transférée à ladite étape de distillation.

6. Procédé suivant l'une quelconque des revendications 1 à 5, **caractérisé en ce qu'**il comprend en outre, après ladite étape de distillation, une pervaporation de ladite phase éthanolique distillée comportant une vaporisation d'éthanol au travers d'au moins une membrane et une rétention d'eau par ladite membrane.

7. Procédé suivant l'une quelconque des revendications 1 à 6, **caractérisé en ce qu'**il comprend, avant ou pendant la digestion, une dilution par de l'eau de la suspension de matière organique issue de l'étape de séparation.

8. Procédé suivant la revendication 7, **caractérisé en ce que** l'eau de dilution est issue de l'étape de distillation et/ou d'une étape de pervaporation de la phase éthanolique distillée.

9. Procédé suivant l'une quelconque des revendications 1 à 8, **caractérisé en ce qu'**il comprend, avant la fermentation, un broyage des matières végétales.

10. Procédé suivant l'une quelconque des revendications 1 à 9, **caractérisé en ce qu'**il comprend, avant la fermentation, une hydrolyse des matières végétales.

## Patentansprüche

1. Verfahren zur Herstellung von biologischem Brennstoff, das Folgendes umfasst:
- eine anaerobe Zersetzung von organischem Stoff mit Bildung eines Biogases, und
- eine Erzeugung von Elektrizität und Wärme durch Verbrennung des Biogases mit zumindest partieller Entnahme von dieser Elektrizität zur Versorgung von mindestens einem Schritt des Verfahrens, der Elektrizität verbraucht, mit Energie, und von dieser Wärme, um sie an mindestens einen Schritt des Verfahrens zu übertragen, der Wärme verbraucht,
**dadurch gekennzeichnet, dass** es ferner Folgendes umfasst:
- eine ethanolische Gärung pflanzlicher Stoffe mit Knollen und/oder Krautpflanzen und/oder pflanzlichen Abfällen aus landwirtschaftlichen Kulturen,
- eine Trennung zwischen einer Suspension organischer Stoffe, die der anaeroben Zersetzung zu unterziehen sind, und einer wässrigen flüssigen Phase, die Ethanol enthält, ausgehend von den gegärten pflanzlichen Stoffen, und
- eine Destillation der wässrigen flüssigen Phase, die Ethanol enthält, mit getrennter Gewinnung von Wasser und einer ethanolischen Phase, die den erzeugten biologischen Brennstoff bildet,
- wobei die Erzeugung von Elektrizität und Wärme durch Verbrennung einzig des Biogases bei Versorgung sämtlicher Schritte des Verfahrens, die Elektrizität verbrauchen, mit Elektrizität und bei Versorgung sämtlicher Schritte des Verfahrens, die Wärme verbrauchen, mit Wärme stattfindet.

2. Verfahren nach Anspruch 1, **dadurch gekennzeichnet, dass** es keine Einleitung von Leitungswasser in das Verfahren umfasst.

3. Verfahren nach einem der Ansprüche 1 und 2, **dadurch gekennzeichnet, dass** als pflanzliche Stoffe mit Knollen und/oder Krautpflanzen und/oder pflanzlichen Abfällen aus landwirtschaftlichen Kulturen Stoffe genutzt werden, die Zucker, Stärke und/oder Zellulose enthalten, insbesondere Rüben, Rübenköpfe, Kartoffeln, Topinambur, Getreide, insbesondere Weizen und Mais, Brennnesseln, Disteln, Unkraut, Zuckerrohr, Chicorée, Miscanthus, Gräser von Brachland oder ihre Mischungen.

4. Verfahren nach einem der Ansprüche 1 bis 3, **dadurch gekennzeichnet, dass** es zwischen dem Gärungsschritt und dem Destillationsschritt einen Zeitraum der Lagerung der gegärten pflanzlichen Stoffe und/oder der wässrigen flüssigen Phase, die Ethanol enthält, umfasst.

5. Verfahren nach einem der Ansprüche 1 bis 4, **dadurch gekennzeichnet, dass** es ferner eine Exposition der wässrigen flüssigen Phase, die Ethanol enthält, gegenüber einer Umkehrosmose mit Erhalt einer Phase mit Feststoffteilchen, die gegebenenfalls in dem Trennungsschritt rückgeführt wird, und einer gereinigten flüssigen Phase, die Ethanol enthält, die zum Destillationsschritt übertragen wird, umfasst.

6. Verfahren nach einem der Ansprüche 1 bis 5, **dadurch gekennzeichnet, dass** es ferner nach dem Destillationsschritt eine Pervaporation der destillierten ethanolischen Phase umfasst, die eine Verdampfung von Ethanol durch mindestens eine Membran hindurch und eine Rückhaltung von Wasser durch die Membran umfasst.

7. Verfahren nach einem der Ansprüche 1 bis 6, **dadurch gekennzeichnet, dass** es vor oder während der Zersetzung eine Verdünnung der Suspension organischer Stoffe, die aus dem Trennungsschritt stammt, mit Wasser umfasst.

8. Verfahren nach Anspruch 7, **dadurch gekennzeichnet dass** das Verdünnungswasser aus dem Destillationsschritt und/oder einem Schritt zur Pervaporation der destillierten ethanolischen Phase stammt.

9. Verfahren nach einem der Ansprüche 1 bis 8, **dadurch gekennzeichnet, dass** es vor der Gärung ein Zerkleinern der pflanzlichen Stoffe umfasst.

10. Verfahren nach einem der Ansprüche 1 bis 9, **dadurch gekennzeichnet, dass** es vor der Gärung eine Hydrolyse der pflanzlichen Stoffe umfasst.

## Claims

1. A method for detecting a biological fuel, comprising
- an anaerobic digestion of organic material, with formation of a biogas, and
- production of electricity and heat by combustion of the biogas, with at least partial withdrawal of this electricity, for supplying energy to at least one step of the method consuming electricity, and of this heat, for transferring it to at least one step of the method consuming heat,
**characterized in that** it further comprises
- ethanolic fermentation of vegetable materials with tubers and/or grasses and/or vegetable wastes from agricultural cultivations,
- separation, from the aforesaid fermented vegetable materials, between a suspension of organic material to be subject to said anaerobic digestion and an aqueous liquid phase containing ethanol, and
- distillation of the aqueous liquid phase containing ethanol with separate collection of water and of an ethanolic phase, which forms the produced biological fuel,
- said production of electricity and of heat only occurring by combustion of said biogas with supply of electricity to all the steps of the method consuming electricity and with supply of heat to all the steps of the method consuming heat.

2. The method according to claim 1, **characterized in that** it does not comprise any introduction of distribution water into the method.

3. The method according to one of claims 1 and 2, **characterized in that**, as vegetable materials with tubers and/or grasses and/or as vegetable wastes of agricultural cultivations, use is made of materials containing sugars, starch and/or cellulose, in particular beets, sugarbeet crowns, potatoes, Jerusalem artichokes, cereals, notably wheat and maize, nettles, thistles, weeds, sugarcane, endives, miscanthus, fallow grasses or mixtures thereof.

4. The method according to any of claims 1 to 3, **characterized in that** it comprises, between said fermentation step and said distillation step, a step for storing said fermented vegetable materials and/or said aqueous liquid phase containing ethanol.

5. The method according to any of claims 1 to 4, **characterized in that** it further comprises exposure to reverse osmosis of said aqueous liquid phase containing ethanol, with obtaining of a phase of solid particles, optionally recycled to said separation step, and of a purified liquid phase containing ethanol which is transferred to said distillation step.

6. The method according to any of claims 1 to 5, **characterized in that** it further comprises, after said distillation step, pervaporation of said distilled ethanolic phase including a vaporizer nation of ethanol through at least one membrane and retention of water by said membrane.

7. The method according to any of claims 1 to 6, **characterized in that** it comprises, before or during digestion, dilution with water of the water of the organic material suspension from the separation step.

8. The method according to claim 7, **characterized in that** the dilution water is from the distillation step and/or a step for pervaporation of the distilled ethanolic phase.

9. The method according to any of claims 1 to 8, **characterized in that** it comprises, before fermentation, milling of the vegetable materials.

10. The method according to any of claims 1 to 9, **characterized in that** it comprises, before fermentation, hydrolysis of the vegetable materials.
